# EUROPEAN PATENT SPECIFICATION

(11) **EP 1 560 024 B1**
(45) Date of publication and mention of the grant of the patent: **07.05.2008**
(21) Application number: 03738136.5
(22) Date of filing: 30.06.2003
(51) Int. Cl.: G01N 33/68

(54) **METHOD OF DIAGNOSING CHANGES IN THE INTESTINAL ABSORPTIVE SURFACE IN AN INDIVIDUAL**
VERFAHREN ZUR DIAGNOSE VON ÄNDERUNGEN DER DARMABSORPTIONSOBERFLÄCHE IN EINEM INDIVIDUUM
METHODE DE DIAGNOSTIC DE L'EVOLUTION DE LA MASSE INTESTINALE ABSORBANTE CHEZ UN INDIVIDU

(30) Priority: 01.07.2002 ES 200201609
(43) Date of publication of application: 03.08.2005
(73) Proprietor: Universidad de Barcelona, 08028 Barcelona (ES)
(72) Inventor: LOPEZ TEJERO, Maria Dolores, 08028 Barcelona (ES); TRAVES POLO, Maria Carmen, 08028 Barcelona (ES)
(74) Representative: ABG Patentes, S.L.
(86) International application number: PCT/ES2003/000323
(87) International publication number: WO 2004/003566

(56) References cited:
- WO-A1-97/10508
- WO-A1-02/092633
- WO-A2-03/008972
- ES-A1- 2 126 512
- STANKOVIC IVAN ET AL.: 'Determination of antisecalin antibodies in sera from coeliac patients by elisc-bared assay' JOURNAL OF PHARMACEUTICAL AND BIOMEDICAL ANALYSIS vol. 18, 1998, pages 255 - 260, XP002987685

## Description

This invention refers to new diagnostic methods and the use of kits in the field of clinical gastroenterology, which are based on the assesment of a natural intestinal protein in a sample of plasma and/or serum removed from a patient.

### BACKGROUND ART

Several intestine function disorders which are totally or partially caused by absence (e.g. because a loss or a lack of maturity) of functionally effective absorptive intestinal mass are malabsorption, coeliac disease, villus atrophy, short bowel syndrome, chronic diarrhea and flatulence, being malabsorption one of the most prevalent ones. Malabsorption (i.e. impaired intestinal absorption of nutrients, mainly sugars and lipids) is a patology associated with several physiological conditions, such as malnutrition, aerophagia, gastrointestinal disturbances and general disconfort. One of the main causes of malabsorption is intestinal atrophy or decrease of intestinal mucosa surface (i.e. decrease of the whole absorptive mass of intestinal villi).

In relation with therapy of different intestine function disorders, it is interesting to diagnose the status of whole functionally effective absorptive mass of a given patient, either at various stages of his individual development (i.e. at his various ages), or along a therapeutical treatment of intestinal failure condition. However, most diagnostic methods used today for this purpose are aggressive and based either on endoscopy or on analysis of jejunal biopsy specimens.

Most clinicians diagnose malabsorption syndromes by endoscopy and intestinal biopsy, but these practices have some drawbacks and limitations. Thus, on several occasions intestinal biopsy is not representative of the status of the whole intestine and a mucosa mosaic is observed with areas of normal and atrophied villi (cf. S.C. Tawil et al., "Scalloping of the valvulae conniventes and mosaic mucosa in tropical sprue"; Gastrointest. Endosc. 1991, vol. 37, pp. 365-367). Besides, the orientation of biopsy may occasionally hinder diagnosis, as a result of which partial intestinal atrophy may not be detected (cf. M. Cassaro et al., "The dark side of the gastric biopsy", Hum. Pathol. 1999, vol. 30, pp. 741-744). About half of those who suffer from celiac diseases (nutritional intolerance to gluten) are not diagnosed since they do not show characteristic symptoms of malabsorption; and the same occurs with tropical sprue (tropical stomatitis). Chromoendoscopic studies, which show magnifications of the intestinal mucosa during endoscopy, reveal that a significant number of partial atrophies would not have been detected by classic endoscopy (cf. L.M. Siegel et al., "Evaluation of latino expatriates for evident of tropical sprue (abstract)"; Gastroenterology 1995, vol. 108, p. A324). Therefore, it would be useful to find serum and/or plasma markers that indicate the clinic status of the intestinal mucosa, particularly intestinal atrophy, and thus obviate the general use of invasive methods like jejunal biopsy and endoscopy.

In clinical field, in order to diagnose intestinal malabsorption, a few methods in serum and/or plasma different from classic biopsy and endoscopy have been suggested in the art. According to one of these methods, lipid malabsorption in patients with chronic pancreatitis is diagnosed by an indirect test that measures the apolipoprotein B-48 (apoB-48) in plasma after a meal rich in lipids (cf. B. Saviana et al., "Diagnosis of lipid malabsorption in patients with chronic pancreatitis: a new indirect test using postprandial plasma apolipoprotein B-48", Am. J. Gastroenterol. 1999, vol. 94, pp. 3229-3235). This method implies the previous isolation of lipoproteins, what is a source of limitations for clinical practice (high volume of blood sample, special and expensive equipment, loss of said lipoproteins during quilomicra isolation, etc.). Another method is based on the measurement of circulating citrulline by HPLC (high performance liquid chromatography) as a marker of malabsorption in cases of short-bowel syndrome (cf. P. Crenn et al., "Postabsortive plasma citrulline concentration is a marker of absorptive enterocyte mass and intestinal failure in humans"; Gastroenterology 2000, vol. 119, pp. 1496-1505). A major limitation of this method is that it can be used only with adults, because the onset for citrulline synthesis in the enterocyte takes place after weaning of mammals (cf. G. Wu et al., "Synthesis of citrulline from glutamine in pig enterocytes"; Biochemical J. 1994, vol. 299, pp. 115-121). Measurement of sugar in urine and serum by HPLC following an oral load of these sugars (lactulose, mannitol, lactose, D-xylose ...) has also been proposed for studying malabsorption in adults and children (cf. S. Travis et al.; "Intestinal permeability: functional assessment and significance"; Clin. Sci. 1992, vol. 82, pp. 471-488. J.A. Kynaston et al., "Simultaneous quantification of mannitol, 3-O-methyl glucose, and lactulose in urine by HPLC with pulsed electrochemical detection, for use in studies of intestinal permeability"; Clin. Chem. 1993, vol. 39, pp. 453-456). But it is frequently annoying for the patient, and it is besides depending of other factors such as gastric emptying and the rate of urine recollection. With paediatric patients, other more indirect methods used, which are also annoying and besides give false positives, are e.g. the breath tests which involve measurement of expired hydrogen produced by bacteria that ferment non absorbed hydrocarbons, or measurement of expired radiactive ¹⁴CO₂ produced by the metabolism of the ¹⁴C-trioleine ingested, which is related to malabsorption of lipids (cf. F. Fernández-Banñares et al., "Procedimientos diagnósticos en gastroenterologia y hepatologia", in P. Humbert & M.A. Gassull (eds.), Capitulo 8, pp. 71-79; Mosby-Dioma Libros S.A.; Madrid 1995).

Thus, it is of clinic interest to find new diagnostic methods that, in a given patient, indicate the status of the whole functionally active absorptive intestinal mass, particularly in relation to several intestine function disorders.

### SUMMARY OF THE INVENTION

According to an aspect of the present invention, it is provided a method of indicating an intestine function disorder caused by an abnormal level of functionally effective absorptive total mass in a given patient, comprising submitting serum and/or plasma samples from patient's blood to an immunoassay for assessing the amount of human apolipoprotein apoA-IV. The immunoassay involves an specific antibody anti-human apoA-IV obtained from a purified intestinal human apoA-IV, a recombinant human apoA-IV or an active fragment of human apoA-IV.

Apolipoprotein A-IV (apoA-IV) is an approximatedly 46 KDa protein synthesised by enterocytes (the most abundant cells of intestinal epithelium), it is released to the lymph bound to chylomicra (lipoproteins synthesised after a meal rich in lipids), and thence it is released to the bloodstream where it is present mainly in free form. ApoA-IV is expressed in human from week 20 of gestation, what means that it can be quantified at birth and prenatal ages.

There are presently eight known isoforms (genetically determined variants) of human apoA-IV. Depending on the population examined, the allele frequency of the primary isoform is about 94 %; the second most prevalent isoform is about 6 %. The other identified isoforms having a very low frequency. By apoA-IV it is here meant any of its isoforms or their mixtures.

The method of the present invention makes use of known assays for detection and quantification of human apoA-IV in serum and/or plasma. Immunodiffusion assays by electrophoresis, such as those disclosed in EP 282.412 B1 for other apolipoproteins, can also be used in the method of the present invention. However, ELISA (Enzyme-linked immunosorbent assays) and Western blot assays are preferred.

The absorptive intestinal mass which is functionally effective in the patient refers to the part of its intestinal mucosa which is mainly formed by enterocytes (absorptive cells). An abnormal level of functionally effective absorptive intestinal mass may be the result of a loss of enterocytes (e.g. by ethanol intake), or may be caused by a lack of maturity of the cells (e.g. in premature neonates). By specific antibody anti-human apoA-IV it is meant any monoclonal or polyclonal antibody which interacts only with human intestinal apolipoprotein apoA-IV. This antibody can be obtained by any of the standard methods known by persons skilled in the art, using as immunogen either purified human apoA-IV (in any of its isoforms or mixtures thereof), or a recombinant human apoA-IV, or an active fragment of human apoA-IV.

In a preferred embodiment, the immunoassay used in the method of the present invention is of the ELISA sandwich type. In a more preferred embodiment, the ELISA sandwich immunoassay involves the pretreatment of the sample with a specific buffer (clearing buffer) containing a pancreatic cholesterol esterase for decreasing interference of sample circulating lipids in apoA-IV quantification. In another preferred embodiment the immunoassay is a Western blot assay (specially preferred with an internal control). And in another preferred embodiment, the immunoassay is an immunodiffusion assay.

Among the intestine function disorders caused by an abnormal level of functionally effective absorptive intestinal mass, which can be diagnosed by the method of the present invention, there are malapsorption, celiac disease (i.e. intolerance to gluten of the diet), intestine villus atrophy, short bowel syndrome, chronic diarrhea and flatulence. Among the different causes of these intestinal disorders are: genetic predisposition, malnutrition, fasted state, parenteral nutrition, intestinal surgery, chemotherapy, premature birth, intestinal bacterial infection, etc.

According to another aspect of the present invention, it relates to the use of a kit for diagnosing an intestine function disorder caused by an abnormal level of functionally effective absorptive intestinal mass (including any of the above-mentioned conditions) in a given patient. The kit has an specific antibody anti-human apoA-IV obtained from a purified intestinal human apoA-IV, from a recombinant human apoA-IV or from an active fragment of human apoA-IV, together with other materials and/or components for assessing the amount of human apolipoprotein apoA-IV by immunoassay. In preferred embodiments, the kit has materials and/or components for an ELISA type immunoassay, or for Western blot immunoassay, or for immunodiffusion assay. For a person skilled in the art it will be easy to select materials and/or components for preparing in vitro kits appropriate for the specific immunoassay, e.g. a microplate with wells in an ELISA, or a film covered with gel in a Western blot or in an immunodifussion assay.

The assessment of the amount of human apolipoprotein apoA-IV can be more or less quantitative depending on the kind of immunoassay used and the availability of pure human apoA-IV as standard, as illustrated in the accompanying examples.

It is noteworthy that several factors such as dietary fat intake, fat malabsorption associated to pancreatic disorders, acute inflammation, and hormonal dysregulation can disturb the plasma apoA-IV concentration in humans. Thus, there are few pathologies associated with lipid metabolism in which these plasma concentrations are altered. In patients with these pathologies the skilled person should use the method of the present invention carefully, taking the blood sample at least after overnight fast. Although absolute levels of apoA-IV in patients with these pathologies can differ significantly from average values of the control population, the evolution of the apoA-IV level for a given patient will still be meaningful. Among these pathologies are the following: coronary disease, obesity, diabetes mellitus, renal insufficiency, chronic pancreatitis and severe undernourishment.

So far human apoA-IV has received little attention in the fields of clinic diagnosis and therapy, an exception being the suggestion of administering human apoA-IV to patients as an injectable formulation for the purpose of controlling appetite and reducing food intake and body weight (cf. WO 94/27629 A1).

Compared with the clinical methods current in the art (endoscopy and jeyunal biopsy), the invention is advantageous in that it provides a diagnostic method which is not invasive to the patient, and that can be easily industrialized with samples of plasma and/or serum removed from the patient.

Compared with the possible assessment of other intestinal proteins, another advantage of this invention arises from the fact that human apoA-IV can be assessed at any patient age of the individual, including prenatal stages. The fact that half life of human apoA-IV is low and its concentration in plasma significantly correlates with its release rate from enterocyte, not with its degradation rate, makes the assessment of human apoA-IV very indicative of the mucosa functional and morphological status. A further advantage, compared with the assessment of other apolipoproteins, arises from the fact that human apoA-IV is released to blood stream mostly in a free form (i.e. non-associated to lipoproteins), what results in that the method of the present invention suffers less from interferences with circulating lipids.

Throughout the description and claims the word "comprise" and its variations are not intended to exclude other additives, components, integers or steps. The disclosure in the abstract is incorporated herein as reference. The following detailed description and drawings are provided by way of illustration, and they are not intended to be limiting of the present invention.

### BRIEF DESCRIPTION OF THE DRAWINGS

FIG. 1 illustrates the detection of human apoA-IV in plasma and intestinal biopsy by Western blot assay.
FIG. 2 shows a histological immunolocalisation of apoA-IV in a human intestinal biopsy of duodenum-proximal jejunum.
FIG. 3 includes two graphs of an ELISA sandwich for detecting apoA-IV in human plasma.
FIG. 4 corresponds to two Western blot assays for purified apoA-IV and human plasma, throughout pre and postnatal development.
FIG. 5 shows apoA-IV levels by ELISA sandwich assay in umbilical cord serum of human neonates, from alcoholic and control mothers, according to the distribution's body weight.
FIG. 6 corresponds to a Western blot assay to detect apoA-IV in umbilical cord serum of human neonates, from alcoholic and control mothers.
FIG. 7 shows detection and quantification by Western blot assay of plasma apoA-IV in coeliac children at different moments of their disease.
FIG. 8 shows detection and quantification by Western blot assay of plasma apoA-IV, in parenterally nourished patients after abdominal surgery.
FIG. 9 shows detection and quantification by Western blot assay of plasma apoA-IV in successive parenteral-(fast state) and enterally nourished patients after gastric surgery.

### DETAILED DESCRIPTION OF PARTICULAR EMBODIMENTS

The method of the present invention, by using both ELISA sandwich and Western blot assays, is illustrated bellow in two representative paediatric pathologies with intestinal failures (foetal alcohol effects and coeliac disease) and in two intestine atrophy models in adults (parenteral nutrition and fast-refeed nutrition). In rat model, inventors have previously shown that etanol consumption by the mother during gestation causes intestinal disorders in foetus and new-born (cf. L. Camps et al., "Effects of prenatal exposure to ethanol on intestinal development of rat fetuses"; Journal Pediatric Gastroenterol. Nutr. 1997, vol. 24, pp. 302-311, and references therein), and some clinical cases in humans have also been published (cf. S. Tourtet et al., "Small intestine atresia and abnormal insertion of the umbilicus in a child with fetal alcohol syndrome"; Archives de Pediatrie 1997, vol. 4, pp. 650-652, and references therein). Coeliac disease (intolerance to gluten of the diet) is a well-established pathology of severe or moderate intestinal villous atrophy, for which the definitive diagnosis is done so far through intestinal biopsy. It is well known that any parenteral nutrition and general fasted state develop mucosa disorder with some atrophy of intestinal villi.

### Population groups of the four models

Babies born from mothers who had consumed alcohol during pregnancy were distinguished of babies born from control mothers: A woman was defined as an alcoholic mother if she had taken alcohol during gestation and before delivery, trough an interview and specific tests. The children diagnosed as coeliac had endomysium antibodies in the serum and took a diet including gluten. The coeliac children who had a jejunal biopsy (almost the 80%) had intestinal atrophy in all cases. The age of study was between 1-6 years-old.

Parenteral nutrition was performed on adult and older patients (30-80 years-old) submitted to abdominal surgery (colon resection). All patients were selected according the following criteria: no kidney or liver disease, no peritoneal carcinomastosis or known metastases, no malnutrition, no weight loss, and no metabolic or endocrine disease. After the operation patients receiving parenteral nutrition support, without oral intake until day seven.

Other groups of patients (submitted to gastric resection) and selected with the same criterion were on parenteral nutrition (tree or five days after surgery) and on enteral nutrition (with liquid and soft diet during four days after) successively.

### Clinical samples

Plasmas and/or serums from patients were taken after overnight fast, except for neonates. At birth serum samples from umbilical cord blood were collected in corresponding Obstetrics and Gynaecology Departments. Plasmas were obtained in disodium EDTA and then centrifuged and frozen at -30° C.

A small fragment of intestinal biopsy was placed at 4° C in PBS (Phosphate Buffer Saline, pH 7.5) and processed as quickly as possible for Western blot and/or histological immunolocalisation studies. Biopsies for western analysis were processed previously at 4°C and to a dilution of 1/25 with PBS-Tritonx100 at 1% (v/v).

Clinical studies were performed with agreement of Ethical Committees of different Hospitals involved: Hospital Clinic de Barcelona, Hospital Sant Joan de Déu in Barcelona, and Hospital Vall d'Hebron in Barcelona.

### Western blot to detect apoA-IV in plasma, serum and intestinal biopsy

The Western blot assays to detect apoA-IV used the proteins separated in 10% SDS-PAGE (sodium dodecylsulfate - polyacrylamide gradient electrophoresis) and transferred on membranes of "Immovilon-P", Millipore®. Aliquots of diluted serum and/or plasma (1/100) were loaded with sample buffer, resulting 0.2 µl of serum and/or plasma per well. The anti-human apoA-IV IgG (developed in inventors' laboratory) obtained from rabbit at 1/500 dilution was used as primary antibody; and anti-IgG from commercial rabbit obtained in pig (marked with horse radish peroxidase, HRP) at 1/15,000 dilution, as secondary antibody (Dako®, Glostrup, Denmark). Other special dilutions for antibodies are described in the corresponding figures. Nonspecific binding was blocked with BSA (bovine serum albumine) at 2% (w/v). To develop the Western blot the ECL (enhanced chemiluminiscence luminol) commercial system was used (Amersham Pharmacia Biotech, Little Chalfont, Buckinghamshire, England) or SuperSignal West Pico chemiluminescent substrate for detection HRP (Pierce®, Rockford, Illinois, USA).

### ELISA sandwich for quantification of apoA-IV in serum and/or plasma

ApoA-IV plasma concentration was determined by an ELISA (enzyme-linked immuno-absorbent assay ) sandwich developed in inventors laboratory. 500 ng of the antibody capture developed in inventors' laboratory (IgG of anti-apoA-IV), obtained in rabbit and diluted in 100 µl of carbonate-bicarbonate buffer (6.9 g/l Na₂CO₃, 10.5 g/l NaHCO₃, pH 9.6), was immunoassayed on a polystyrene plate with 96 wells. After all-night incubation of the plate at 4 °C in a humid chamber, it was washed five times with PBS-T (Phosphate Buffer Saline pH 7.4, with 0.05 % Tween-20, v/v) at room temperature. To block nonspecific binding, each well was incubated with 360 µl of blocking solution containing bovine serum albumin (BSA) (PBS and 0.5 % BSA, w/v) for one hour at 37°C in a humid chamber and shaken. After being washed five times with PBS-T, 100 µl of the samples, the standard and the internal control (for inter- and intra-assays, see below paragraph) already diluted in PBS-T-BSA (PBS, 0.5 % BSA and 0.05 % Tween-20), were incubated for one hour at 37 °C in a humid chamber. Then it was washed five more times with PSB-T.

The plate was then incubated for 2 hours at room temperature in the humid chamber with the antibody detector, i.e. the same human IgG apoA-IV antibodies obtained in rabbit, but bound to biotine (0.5 mg/ml, 12.4 mol biotine/mol lgG), and diluted 1/500 in 100 µl PBS-T-BSA. When the incubation was complete, the plate was washed five times with PSB-T. Then it was incubated for 20 min at room temperature in the humid chamber with a solution of avidine bound to 0.2 mg/l peroxidase and diluted in PBS-T-BSA (100 µl per well). It was washed five times with PSB-T solution. Then 100 µl per well of OPD solution (0.4 g/l O-phenylendiamine, 7.3 g/l citric acid, 9.6 g/l Na₂HPO₄ and 0.2 µl/ml H₂O₂), prepared at moment of use, was pipetted. The development or appearance of the product's colour took place at room temperature. After 20 min at most, the reaction was stopped by adding 50 µl per well of 2.5 M HCl. The plate was shaken for a few seconds in an orbital shaker and was read at 492 nm with the "Titertek Multiskan" ELISA reader.

Pre-treatment of plasmas or serums: In pilot experiments to fine-tune ELISA not totally satisfactory results were obtained: There was no linearity in the volume of the samples; and inter- and intra-assay repetition was unacceptable. The samples had to be pre-treated with pancreatic cholesterol esterase, avoiding possible interference of plasma lipids in quantification of apoA-IV, in order to attain better results. The plasma samples and the internal control (for inter- and intra-assays) were diluted 1/10 in PBS and then incubated at 37°C with clearing buffer used for nephelometry which contained: 87.2 g/I aspartic acid, 17.5 g/I sodium cholate, 1.2 % Tween 20, 0.4 g/I cholesterol esterase, and 0.1 M Tris, pH 7.7 in (1:1) (v:v). The reaction was halted with ice after ten minutes and then PBS-T-BSA was added, until the final dilution of the sample (1/150 or 1/300) was attained. Clearing buffer was processed without serum and/or plasmas as background. The standard was made from an apoA-IV solution previously purified in inventors laboratory. Protein concentration was confirmed with the commercial kit "BCA Protein assay reagent" (Pierce®, Rockford, Illinois), using BSA as standard. The standard of apoA-IV was diluted in PST-T-BSA, using a concentration range of 1 to 2,000 ng per well. It was established that there was no interference of the clearing buffer in the standard, and therefore it was not used in it.

The standard curve was adjusted through polynomial regression analysis. Two dilutions of each sample were assayed (1/150 and 1/300) and the apoA-IV concentration was calculated from the mean of the two dilutions. The dilutions of the sample and the various concentrations of the standard control were assayed in duplicate. The internal control was an adult plasma overnight fasted with a concentration of apoA-IV: 13.14 mg/100 ml, and it was assayed in quadruplicate. The intra- and inter-assay was respectively 4.2 % and 9.1 %.

### Detection of human apoA-IV in plasma and intestinal biopsy by Western blot assay.

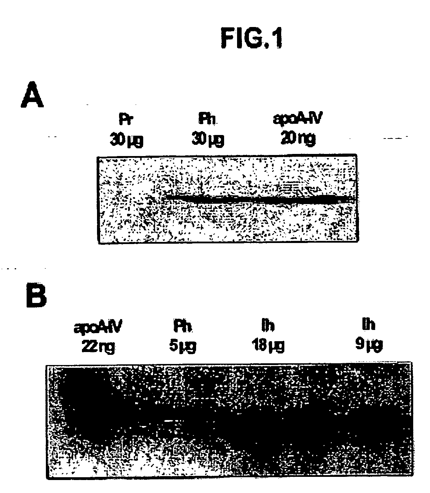

The specificity of antibodies (from rabbit IgG) obtained by inventors, for detecting apolipoprotein apoA-IV in human plasma and intestinal biopsy, is illustrated by results in FIGs. 1,2 and 3.

FIG. 1 illustrates results obtained by SDS-PAGE electrophoresis and Western blot. In FIG. A 30 pg of protein from plasmas (rat and human origin) were loaded in wells. A well was loaded with 20 ng of purified human apoA-IV. The primary antibody was diluted 1/200, and the secondary antibody (anti rabbit IgG bound to HRP, horse radish peroxidase) to 1/2,000. Detection was made by DAB (diaminobenzidine). It was demonstrated that the primary antibody do not label the apoA-IV from rat. In FIG. B 22 ng of purified human apoA-IV and 5 pg of protein of human plasma were loaded. Different protein contents of intestinal biopsy (18 µg and 9 µg) were loaded in different wells. The primary and secondary antibodies were processed identically to results of FIG. A. Detection was made by ECL. Detection by ECL showed higher label than detection by DAB. FIG. 1A symbols are as follows: Pr= plasma from rat. Ph= plasma from human. ApoA-IV is the protein purified in inventors' laboratory. FIG. 1B symbols are as follows. Ih means human intestinal biopsy (from duodenum-proximal jejunum).

### Immunolocalisation of apolipoprotein A-IV in a human intestinal biopsy of duodenum-proximal jejunum.

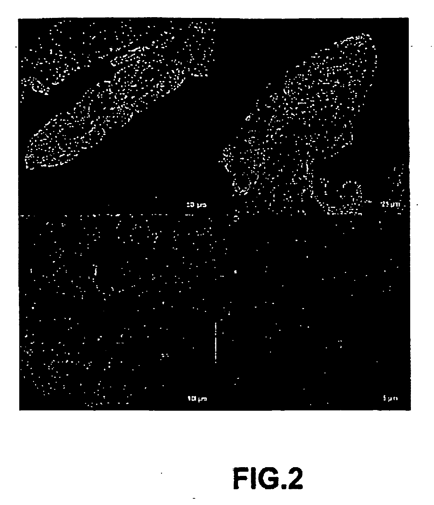

FIG. 2 shows immunolocalisation of apoA-IV in human intestine using confocal microscopy (Leica TCS 4D, Serveis Cientifico-Tècnics, Parc Cientific, UB). The primary antibody (rabbit IgG) was diluted to 1/40 in PBS-FCS (fetal calf serum at 10 %, v/v); as secondary antibody it was used anti rabbit IgG obtained from goat bound to Texas red which was diluted 1/200 in PBS-FCS. The images showed that the protein was located along the epithelium of the intestinal mucosa (see to 50 and 25 µm), and specifically in enterocytes with no blot found in other kind of cells. ApoA-IV was mainly located in the supranuclear area of the enterocyte (see to 10 and 5 µm). It was shown the cellular location and specificity of inventors antibody (rabbit IgG) for detecting human apoA-IV synthesised basically by enterocytes. These data supplemented previous results obtained with Western blot assay in FIG. 1B.

### ELISA sandwich for detecting apolipoprotein A-IV in human plasma.

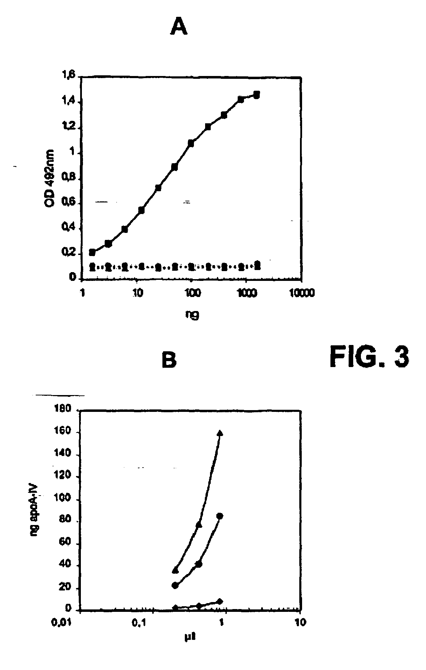

FIG. 3 shows different results of ELISA sandwich method. FIG. 3A illustrates the specificity of inventors antibody for detecting growing concentrations of purified human apoA-IV. Commercialised human apoA-I (synthesised also by the intestinal mucosa) and human albumin are not detected using the same ELISA sandwich method. In FIG. 3B increasing volumes of sample (dilution assayed) are correlated to human plasmas with varying amounts of apoA-IV: low levels for neonates, high levels for a child of 5-6 years-old, and adult levels for an adult of 20 years-old. The recuperation value of purified apoA-IV into different samples was between 90.5 and 106.2 %. FIG. 3A symbols are as follows: human apoA-IV (squares), human apoA-I (triangles) and human albumin (circles). FIG. 3B symbols are as follows: plasma from a child of 5-6 years-old (triangles), plasma from an adult of 20 years-old (circles), plasma from a neonate birth at term (rhombus).

### Western blot assay for purified apoA-IV and for detecting apoA-IV in human plasma throughout pre and postnatal development.

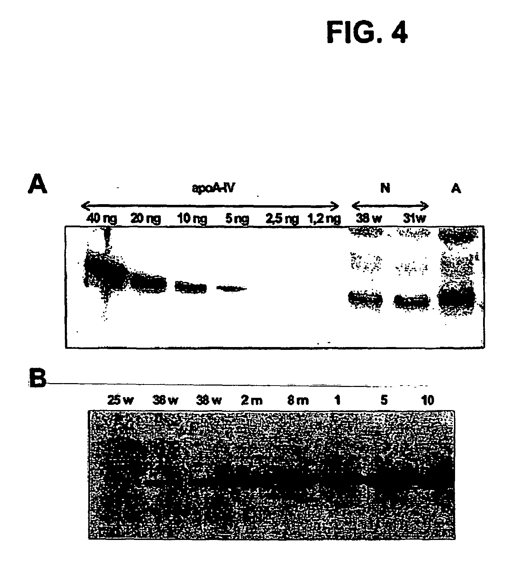

FIG. 4 shows different blots of human plasma with different amounts of apoA-IV according the stage of development (from birth to adult ages). Western blot assay can be used as an alternative method to ELISA sandwich quantification. Detection limit of Western blot in the present experimental conditions is about 5-2 ng of protein apoA-IV and 0.2 µl of serum and/or plasma sample. Correlation index of values from 59 samples parallel measured by both analysis was 0.841. Although the Western blot assay is a semi-quantitative technique comparatively to ELISA, its main advantage is the absence of interference of plasma lipids. FIG 4A symbols are as follows. N: umbilical cord serum of neonates (38 and 31 weeks of gestation); A: plasma of an adult (20 years-old). FIG. 4B refers to plasma from different times of human gestation (25 weeks, 36 weeks and 38 weeks); nursing babies from different periods of lactation (2 and 8 months-old) and children of different ages (1, 5 and 10 years-old).

### ApoA-IV levels in umbilical cord serum of human neonates from alcoholic and control mothers according to the distribution's body weight.

TABLE 1 shows body weight, and levels of apoA-IV, triacylglycerides, and cholesterol in umbilical cord serum of neonates of 36 to 42 weeks gestation. ApoA-IV of these samples was quantified through ELISA sandwich (FIG. 3). To determine triacylglycerides and cholesterol in serum, the commercial kits were used (Medical Analysis Systems and Boehringer Mannheim GmbH respectively). Ethanol in utero significantly reduced the apoA-IV in umbilical cord serum at birth, regardless of the neonate's body weight. This result could be related with morphological and functional disorders of the intestine found by the inventors in the experimental foetus (from rat) affected by alcohol ingested by the mother, probably linked to ethanol present in amniotic fluid and intestinal lumen of the foetus during gestation. Data give mean ± SEM of n individual. The Mann-Whitney U test was applied. Neonates C= neonates of control mothers. Neonates A= neonates of alcoholic mothers. NS= not significant.

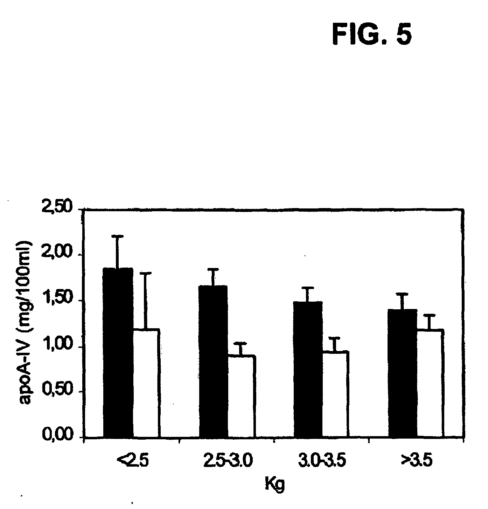

FIG. 5 presents data for apoA-IV in serum (TABLE 1) according to neonates' weight distribution. Neonates from alcoholic mothers always had less apoA-IV than control neonates (specially in neonates weighing between 2.5 and 3.5 Kg). Data give mean ± SEM of n individual. Filled bars = neonates of control mothers. Open bars = neonates of alcoholic mothers.

**TABLE 1: Body weight, apoA-IV, triacylglycerides, and cholesterol in umbilical cord serum of neonates of 36 to 42 weeks gestation.**

| | Neonates C | Neonates A | *p*-Values |
|---|---|---|---|
| | n=139 | n=78 | |
| Body weight (g) | 3140±29 | 3220±32 | NS |
| ApoA-IV (mg/100ml) | 1.66±0.09 | 1.14±0.11 | <0.001 |
| triacylglycerides (mg/100ml) | 40.43±1.51 | 41.22±2.17 | NS |
| Cholesterol (mM) | 1.75±0.04 | 1.78±0.05 | NS |

### Western blot assay to detect apoA-IV in umbilical cord serum of human neonates from alcoholic and control mothers.

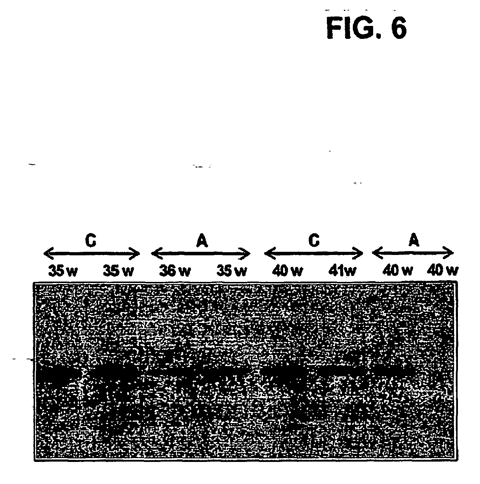

FIG. 6 shows a Western blot of apoA-IV analysing serum of some neonates babies of control and alcoholic mothers with different lengths of gestation. It is observed that the blot intensity in serum of offspring from alcoholic group is less detectable than in serum of control neonates with the same length of gestation. These results complement data of TABLE 1 and FIG. 5. Cord serum in neonates of 35, 36, 40 and 41 weeks gestation. C = neonates from control mothers. A = neonates from alcoholic mothers.

### ApoA-IV levels in plasma of coeliac children

TABLE 2 shows the concentration in plasma of apoA-IV, triacylglycerides and cholesterol in active coeliac children (with gluten intake) between 1 and 6 years-old and controls of the same age. ApoA-IV of these samples were quantified through ELISA sandwich (FIG. 3). To determine triacylglycerides and cholesterol in plasma, the commercial kits were used (Medical Analysis Systems and Boehringer Mannheim GmbH respectively). Triacylglyceride values were significantly higher in coeliac children than controls. Cholesterol values were significantly lower in coeliac children than controls. ApoA-IV values in coeliac children were below values for controls, although no significant differences were found between groups. Data given are mean ± SEM of n individual. The Mann-Whitney U test was applied. NS= not significant.

**TABLE 2: ApoA-IV, triacylglycerides and cholesterol in plasma of active coeliac children (between 2 and 6 years old) and controls of the same age.**

| | Coeliacs | Controls | *p*-Values |
|---|---|---|---|
| | n=40 | n=173 | |
| ApoA-IV (mg/100ml) | 7.87±0.80 | 9.58±0.70 | NS |
| Triacylglycerides (mg/100ml | 89.53±6.81 | 55.93±2.54 | <0.01 |
| Cholesterol (mM) | 3.23±0.11 | 3.97±0.26 | <0.01 |

### Western blot assay to detect apoA-IV in plasma of coeliac children at different moments of their disease

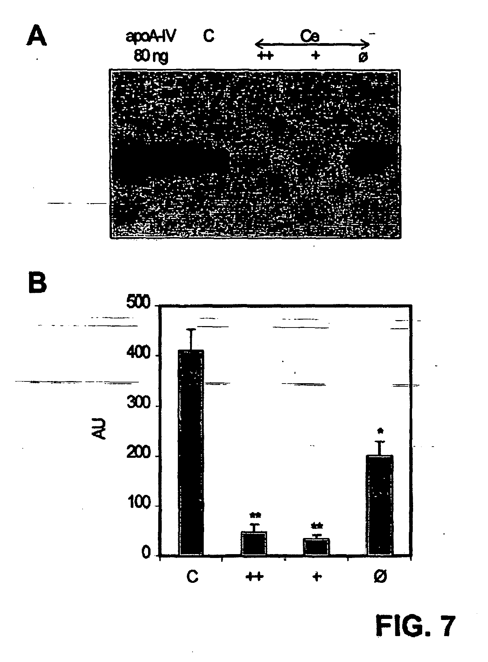

Western blot assay of plasma apoA-IV in coeliac children at different moments of their disease is presented in FIG. 7. In FIG. 7A plasma samples of coeliac and control children and 80ng of purified apoA-IV was loaded. The coeliac child in active phases of their disease [with positive serum markers (+): IgA-AGA and IgA-AEA antibodies] had less intense apoA-IV blot than the same children nourished with a gluten-free diet (Ø), and significant lower than figures for controls of the same age (FIG. 7B) (data as arbitrary units of six given individual). FIG. 7 symbols are as follows. C = control children. Ce = coeliac children. [+ or ++] = the coeliac children in active phases of their disease with positive serum markers ( IgA-AGA and IgA-AEA antibodies). [Ø] = the same children nourished with a gluten-free diet (treated-coeliac). In FIG. 7B arbitrary units are give as the mean±SEM for n=6 individual (C and Ce). The Mann-Whitney U test was applied (p<0.01 **, and p<0.1 * are significant differences versus C).

The lower apoA-IV levels in plasma assayed by Western blot of coeliac children reveals a disorder in the metabolism of this protein, probably related to the atrophy of the intestinal mucosa and malnutrition of these children. Coeliac children aged between 1 and 6 have a disturbance in lipid metabolism characterised by a drop in the plasma concentration of cholesterol and apoA-IV and a rise in plasma triacylglycerides. These disturbances may be directly related to intestinal malabsorption and malnutrition associated with coeliac disease.

### Western Blot assay to detect apoA-IV in plasma of surgical patients parenterally nourished

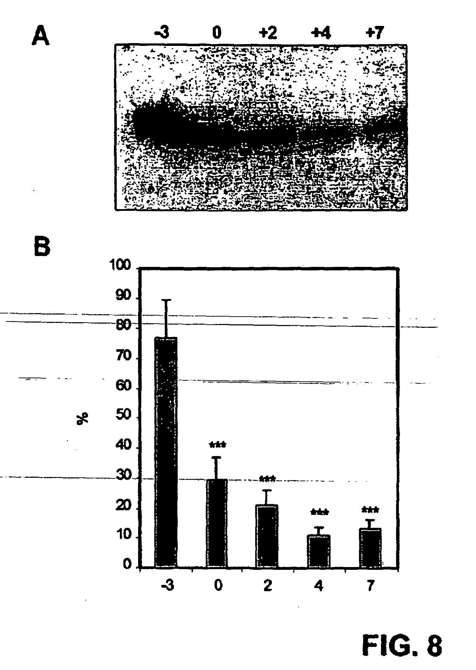

Western Blot assay of plasma apoA-IV in parenterally nourished patients (different sex and age) after abdominal surgery (colon resection) were present in FIG. 8. Plasma samples of every patient were loaded as in FIG. 8A. Days of study were: three days prior to surgery (overnight fasting); during surgery (between 24 and 48 hours minimum of fast); and 2, 4 and 7 days after surgery (receiving parenteral nutrition support). FIG 8B shows arbitrary units quantified as percentage in relation to the blot of an internal control (plasma from a healthy young adult -overnight fasted) loaded in the same gel. These results clearly showed significant, successive and profound changes in the blot of plasma apoA-IV along days of individual submitted to parenteral nutrition. FIG. 8A symbols are as follows: -3 = three days prior to surgery ; 0 = during surgery; +2,+4,+7 = 2, 4 and 7 days after surgery. In FIG. 8B arbitrary units were quantified as percentage in relation to the blot of an internal control. Data give mean ± SEM of n=21 individual. The Wilcoxon test was applied (p<0.001 *** means significant differences versus preoperative values of a given patient, -3).

### Western Blot assay to detect apoA-IV in plasma of surgical patients fasted and in nourished in successive enterally diet

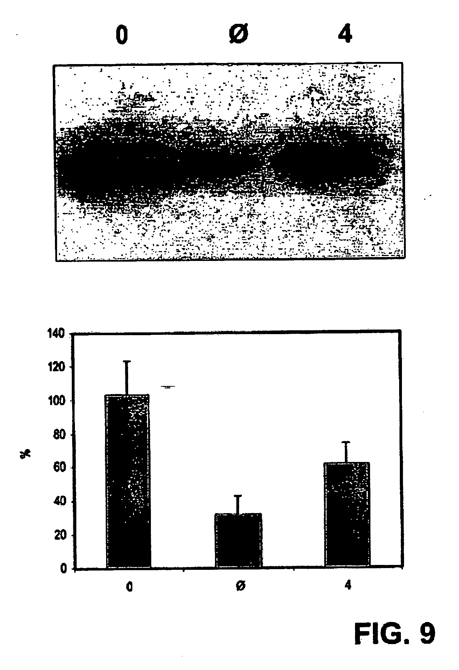

Results in parenteral-enterally nourished patients after abdominal surgery (gastric resection) correspond to data of FIG. 9. FIG. 9 A presents results of Western blot assay of plasma apoA-IV in successive parenteral- and enterally nourished patient. The same criteria of clinical selection was used that for patients of study of FIG. 8. Days of study were: just before surgery (18 hours minimum of fast); just before the onset of oral nutrition (three days minimum of parenteral nutrition); and after four days receiving enteral nutrition (excluded fat diet). FIG. 9B shows arbitrary units quantified as percentage in relation to the blot of an internal control (plasma from a healthy young adult -overnight fasted) loaded in the same gel. These results clearly showed in this group of individual that there is an increase of the blot when diet change of the fast state to enteral nutrition in the same patient. FIG. 9A symbols are as follows: 0 = just before surgery; Ø = just before the onset of oral nutrition and three-four days on parenterally nutrition (fast state); and 4 = four days receiving only enteral nutrition. In FIG. 9B arbitrary units were quantified as percentage in relation to the blot of an internal control. Data give mean ± SEM of n= 6 individual.

## Claims

1. An *in vitro* method to indicate the status of the whole functionally effective absorptive intestinal mass in a given patient, comprising submitting serum and/or plasma samples from a patient to an immunoassay for assessing the amount of human apolipoprotein A-IV (apoA-IV) in said sample.

2. The method according to claim 1, wherein said immunoassay involves an specific antibody anti-human apoA-IV.

3. The method according to claim 2, wherein said antibody is obtained from a protein selected from the group consisting of purified intestinal human apoA-IV, a recombinant human apoA-IV, and an active fragment of human apoA-IV.

4. The method according to claim 1, wherein the immunoassay is of the ELISA sandwich type.

5. The method according to claim 4, wherein the ELISA sandwich immunoassay involves the pretreatment of the sample with a clearing buffer containing pancreatic cholesterol esterase.

6. The method according to claim 1, wherein the immunoassay is a Western blot assay.

7. The method according to claim 1, wherein the immunoassay is an immunodiffusion assay.

8. Use of a kit comprising: (i) an specific antibody anti-human apoA-IV obtained from a protein selected from the group consisting of purified intestinal human apoA-IV, a recombinant human apoA-IV or an active fragment of human apoA-IV; and (ii) other materials and/or components for assessing the amount of human apolipoprotein apoA-IV by immunoassay, for diagnosing the status of the whole functionally effective absorptive intestinal mass in a given patient.

## Patentansprüche

1. In-vitro-Verfahren zur Anzeige des Status der gesamten funktionell effektiven absorptiven Intestinalmasse bei einem gegebenen Patienten, umfassend das Unterwerfen von Serum und/oder Plasmaproben von einem Patienten einem Immunassay zur Bewertung der Menge von menschlichem Apolipoprotein A-IV (apoA-IV) in der Probe.

2. Verfahren gemäß Anspruch 1, wobei der Immunassay einen spezifischen Antikörper Anti-human apoA-IV involviert.

3. Verfahren gemäß Anspruch 2, wobei der Antikörper von einem Protein erhalten wird, ausgewählt aus der Gruppe bestehend aus gereinigtem intestinalem humanem apoA-IV, einem rekombinanten humanen apoA-IV und einem aktiven Fragment von humanem apoA-IV.

4. Verfahren gemäß Anspruch 1, wobei der Immunassay vom ELISA-Sandwichtyp ist.

5. Verfahren gemäß Anspruch 4, wobei der ELISA-Sandwich-Immunassay die Vorbehandlung der Probe mit einem Aufklärungspuffer enthaltend Pankreascholesterinesterase involviert.

6. Verfahren gemäß Anspruch 1, wobei der Immunassay ein Western-Blot-Assay ist.

7. Verfahren gemäß Anspruch 1, wobei der Immunassay ein Immundiffusionsassay ist.

8. Verwendung eines Kits, umfassend: (i) einen spezifischen Antikörper Anti-human apoA-IV, erhalten von einem Protein ausgewählt aus der Gruppe bestehend aus gereinigtem intestinalem humanem apoA-IV, einem rekombinanten humanen apoA-IV oder einem aktiven Fragment von humanem apoA-IV und (ii) andere Materialien und/oder Komponenten zur Bewertung der Menge von menschlichem Apolipoprotein apoA-IV durch Immunassay zur Diagnose des Status der gesamten funktionell effektiven absorptiven Intestinalmasse bei einem gegebenen Patienten.

## Revendications

1. Méthode *in vitro* pour indiquer le statut de la masse intestinale à absorption fonctionnellement efficace totale chez un patient donné, comprenant les étapes consistant à soumettre des échantillons de sérum et/ou de plasma d'un patient à une analyse immunologique pour déterminer la quantité d'apolipoprotéine A-IV humaine (apoA-IV) dans ledit échantillon.

2. Méthode suivant la revendication 1, dans laquelle ladite analyse immunologique fait intervenir un anticorps spécifique anti-apoA-IV humaine.

3. Méthode suivant la revendication 2, dans laquelle ledit anticorps est obtenu à partir d'une protéine choisie dans le groupe consistant en une ApoA-IV humaine intestinale purifiée, une ApoA-IV humaine recombinante et un fragment actif d'ApoA-IV humaine.

4. Méthode suivant la revendication 1, dans laquelle l'analyse immunologique est du type analyse ELISA en sandwich.

5. Méthode suivant la revendication 4, dans laquelle l'analyse immunologique ELISA en sandwich comprend le prétraitement de l'échantillon avec un tampon de clarification contenant de la cholestérol-estérase pancréatique.

6. Méthode suivant la revendication 1, dans laquelle l'analyse immunologique est une analyse de transfert d'empreinte par la méthode de transfert d'empreinte Western.

7. Méthode suivant la revendication 1, dans laquelle l'analyse immunologique est une analyse d'immunodiffusion.

8. Utilisation d'un kit comprenant: (i) un anticorps spécifique anti-ApoA-IV humaine obtenu à partir d'une protéine choisie dans le groupe consistant en une ApoA-IV humaine intestinale purifiée, une ApoA-IV humaine recombinante et un fragment actif d'ApoA-IV humaine ; et (ii) d'autres substances et/ou constituants pour déterminer la quantité d'apoliprotéine ApoA-IV humaine par analyse immunologique, pour le diagnostic du statut de la masse intestinale à absorption fonctionnellement efficace totale chez un patient donné.
